Europäisches Patentamt

(19) European Patent Office

Office européen des brevets

(11) **EP 0 450 152 B1**

(12) # EUROPEAN PATENT SPECIFICATION

(45) Date of publication and mention
of the grant of the patent:
**02.10.1996 Bulletin 1996/40**

(51) Int. Cl.$^6$: **A61B 6/03**, G06F 15/78

(21) Application number: **90121417.1**

(22) Date of filing: **08.11.1990**

(54) **Computerized tomographic imaging method and apparatus utilizing data interpolation for helical scanning**

Verfahren und Vorrichtung zur rechnerunterstützten Erzeugung von Tomographien mittels schraubenförmiger Abtastbewegung

Procédé et dispositif destinés à l'imagerie tomographique à l'aide d'un balayage hélicoidal

(84) Designated Contracting States:
**DE NL**

(30) Priority: **04.04.1990 JP 89774/90**

(43) Date of publication of application:
**09.10.1991 Bulletin 1991/41**

(73) Proprietor: **KABUSHIKI KAISHA TOSHIBA**
**Kawasaki-shi, Kanagawa-ken 210 (JP)**

(72) Inventor: **Toki, Yusuke**
**Utsunomiya-shi Tochigi-ken (JP)**

(74) Representative: **Blumbach, Kramer & Partner**
**Patentanwälte**
**Radeckestrasse 43**
**81245 München (DE)**

(56) References cited:
**EP-A- 0 383 232**     **US-A- 4 086 492**
**US-A- 4 630 202**     **US-A- 4 789 929**

## Description

The present invention relates to an X-ray CT (computerized tomographic) imaging apparatus and method according to the pre-characterizing part of claim 1 and claim 5, respectively. More particularly, the present invention is directed to a method and an apparatus for interpolating projection data acquired during a helical scanning operation by using projection data produced by opposite X-ray beams.

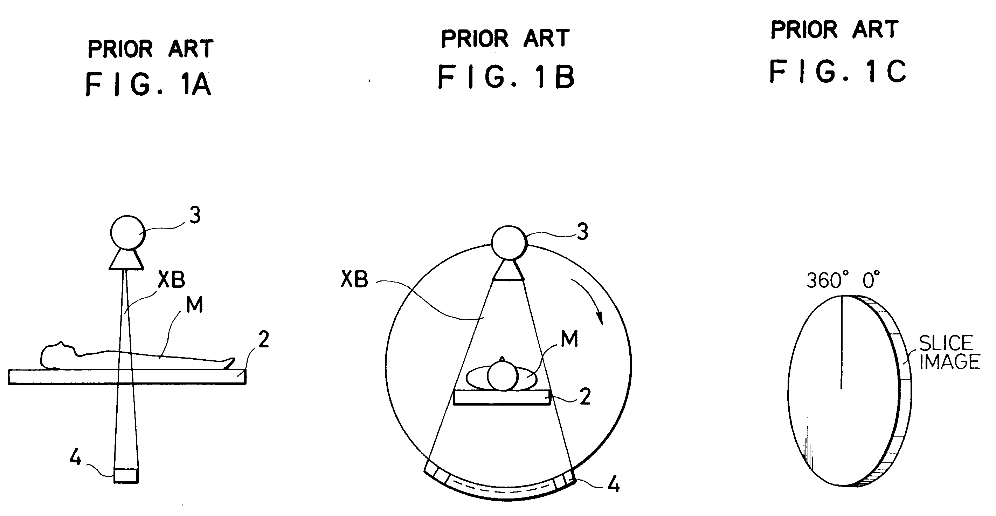

In general, a so-termed "3rd generation type X-ray CT imaging apparatus" is well known in the medical electronic field. The typical X-ray CT imaging system of the 3rd generation will now be summarized. As represented in Figs. 1A and 1B, while an object "M" under medical examination such as a patient is fixedly mounted on a couch 2, and also both an X-ray tube 3 and an X-ray detector 4 are integrally rotated around the object M sandwiching this object M by them, an X-ray beam "XB" generated from the X-ray tube 3 is projected to the object "M" in order to acquire X-ray projection data over 360 degrees around the object M. Based upon the acquired projection data, a predetermined calculation/processing operation is executed so as to reconstruct an image, i.e., a computerized tomographic image (slice image) of the object "M" along the X-ray projection plane. Fig. 1C schematically illustrates an idea of this slice image. Accordingly, the X-ray projection data having the slice width corresponding to the projection width of the X-ray beam have been acquired over 0 degree to 360 degrees and the image reconstruction is carried out to obtain such a slice image.

In such a conventional scanning system while projecting the X-ray beam "XB" to the entire surface of the object "M" under condition that the object "M" is fixed on the couch 2, the projection data are acquired in the range of 360 degrees. When a plurality of tomographic images are to be obtained at the different projection angles of the object "M" at one slice-obtaining position, the scanning operation must be interrupted and the object must be translated along the longitudinal direction of the couch 2 until a subsequent slice-obtaining position after the above-described scanning operation has been reached with respect to the previous slice-obtaining position. Then, a series of such scanning operations is restarted at this subsequent slice-obtaining position. Subsequently, the scanning operations must be similarly carried out with respect to the succeeding slice-obtaining positions. As a consequence, since an entire scanning time involving the patient translating time becomes considerably long in case that a large number of slice images are acquired from the same object, the operation efficiency (i.e., throughput) of the X-ray CT imaging apparatus is lowered. Furthermore, since the total time during which the object under medical examination is forcedly fixed on the couch is similarly prolonged, excessive psychological pressure or pain may be given to the object under medical examination.

To mitigate such a drawback, another conventional X-ray CT imaging system with a specific scanning method has been developed. This improved scanning system is known as "a helical scanning system" from, for instance, US-A 4,630,202. This publication discloses the features mentioned in the preamble of attached claims 1 and 5.

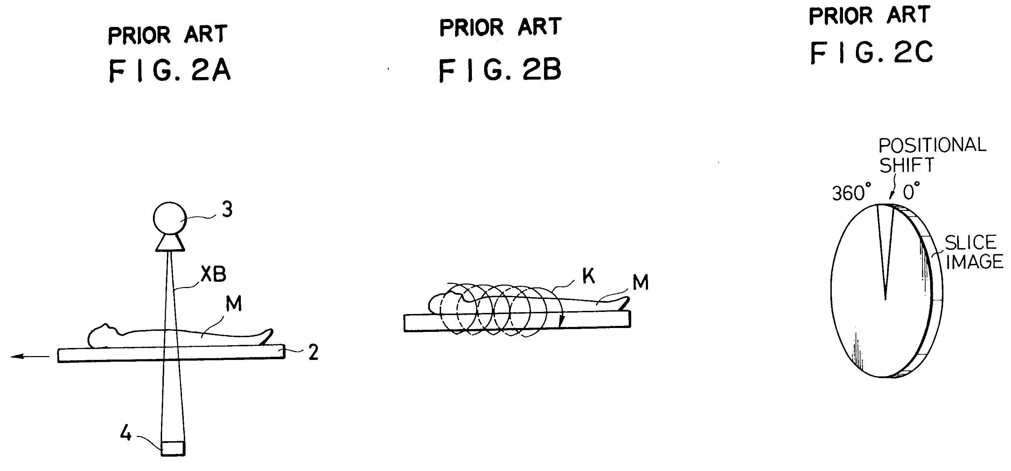

In this conventional helical scanning system, as represented in Fig. 2A, while the couch 2 for supporting the object "M" thereon is moved or translated along an arrow, the X-ray tube 3 is continuously rotated around the object "M" to project the X-ray beam "XB" thereto. Since an X-ray projection path (namely, a trail of the X-ray tube 3) "K", is varied in a helical form with respect to the object "M", as shown in Fig. 2B, this scanning system is referred to as "a helical scanning". In accordance with such a conventional helical scanning system by continuously projecting the X-ray beam to the object, there are various advantages in that the above-described drawback of the first-described conventional X-ray CT imaging apparatus may be overcome and also a large quantity of projection data may be acquired within a single helical scanning operation.

However, another problem may be caused in this helical scanning system. That is, since positions of the object "M" on which the X-ray beams "XB" are incident over one rotation (i.e., 360 degrees) are slightly shifted, the slice positions are always varied and therefore incoincidence may inherently occur in the projection data acquired at 0 degree and 360 degrees due to the above-described positional shifts. Such positional incoincidence occurring in the reconstructed tomographic image is illustrated in Fig. 2C. This fact is unfortunately a deviation from the basic condition of the CT image reconstruction "to acquire projection data in all directions within the same slice plane of an object under medical examination". As a result, an artifact may intensely occur in the obtained tomographic image. Therefore, correct diagnostic information on the object under medical examination may not be expected.

To avoid such difficulties, projection data on arbitrary slice positions are interpolated based upon the actually acquired projection data and thereafter both the interpolated projection data and the actually acquired projection data are processed for an image reconstruction purpose in the above-described conventional helical scanning system.

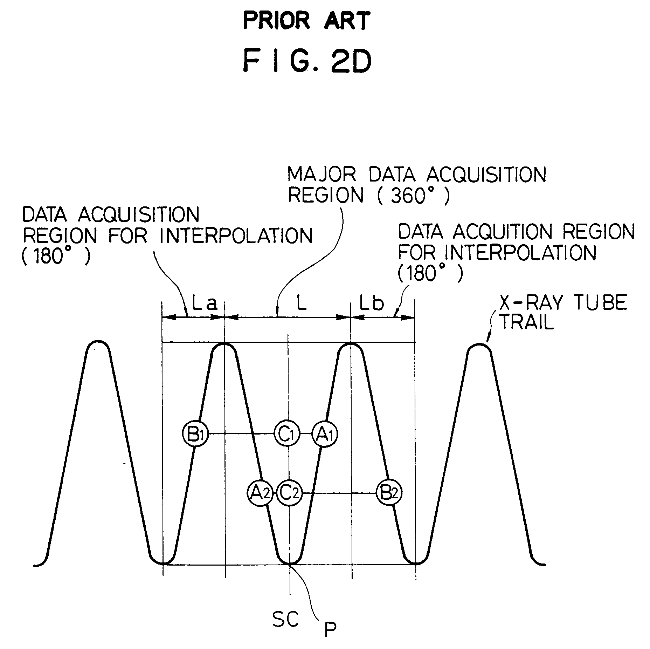

Fig. 2D is a representation for explaining a principle of the interpolation method employed in the conventional helical scanning system (EP-A-0 383 232, published 22.08.90.) Under condition that the X-ray tube trail is varied in a sinusoidal form during the helical scanning operation, a region for obtaining a single slice corresponding to an arbitrary rotation (360 degrees) around the object under medical examination is set as "a major data acquisition region". Assuming now that a center position of this 360-degree data acquisition region is a slice center position "SC", the projection data can be actually acquired only on a point "P", which the X-ray tube trail has intersected, along a center line of this slice center "SC", whereas the projection data cannot be actually acquired from any points along this center line, other than this point "P". As a consequence, if projection data of, for instance, a point "$C_1$" located at an arbitrary position on this center line of the slice center "SC" is required, this projection data cannot be actually acquired but may be calculated by interpolating both actually acquired projection data on a point "$A_1$" and also another point "$B_1$". This point "$A_1$" is positioned at the nearest distance with respect to this point "$C_1$" and the point "$B_1$" is positioned far from this point "$C_1$" but in the same rotation phase with these points $C_1$ and $A_1$. This interpolation is carried out by utilizing the known linear interpolation method, and distances between this point $C_1$ and any points at the same rotation phase are used as weight coefficients for this linear interpolation. For instance, assuming now that a distance of ($A_1$ - $B_1$) is selected to be "1" and another distance of ($A_1$ - $C_1$) is selected to be "$\ell$", desirable projection data "$D_{C1}$" is calculated by way of the following linear interpolation theory based on the below-mentioned distance weight coefficients;

$$D_{C1} = D_{A1} \times (1 - \ell) + D_{B1} \times \ell \qquad (1)$$

where $D_{A1}$ corresponds to projection data on the point $A_1$, and $D_{B1}$ corresponds to projection data on the point $B_1$.

Similarly, when another projection data which cannot be actually acquired, on a point "$C_2$" along the center line of the slice center "SC" is desired, both the actually-acquired projection data at a point "$A_2$" which is located at the shortest distance from this point $C_2$ in the same rotation phase, and also the other actually-acquired projection data at a point "$B_2$" located opposite to this point "$A_2$" in the same rotation phase are employed for the linear interpolation calculation. Since such a data interpolation processing operation is carried out in a similar manner as the above-described method and therefore the interpolated projection data at zero degree is coincident with that at 360 degrees within the 360°-X-ray beam projection range, the above-described occurence of an artifact may be reduced.

When the above-defined linear interpolation is performed, since the projection data actually acquired at predetermined points within both data acquisition regions at the same rotation phase with respect to the center line of the slice position "SC" are utilized, both data acquisition regions "La" and "Lb" for interpolation are necessarily required within each 180-degree range at maximum with respect to the major data acquisition region "L" of 360 degrees. Accordingly, the above-described conventional linear interpolation calculation is performed by utilizing the actually acquired projection data obtained from the data acquisition region of 720 degrees in total.

As previously explained, since the actually acquired projection data are obtained from the data acquisition region of 720 degrees involving the 360-degree data acquisition regions "La" and "Lb" for interpolation purposes, there is a problem that the thickness of the resultant effective slice becomes thick. In other words, the thickness of the calculated effective slice necessarily becomes thick, because the desirable projection data on an arbitrary point along the center line of the slice center "SC" is calculated by linear-interpolating the projection data actually acquired at two points which are located in the same rotation phase with the arbitrary point and within two date acquisition regions with respect to the major data acquisition region involving this slice center "SC". As a consequence, since the actually acquired projection data which have been acquired from the points located relatively far from the slice position must be utilized for this interpolation calculation, the calculated desirable projection data considerably deviate from the true, or actually-acquired projection data, which may cause the interpolation precision to be deteriorated.

The present invention has been made in an attempt to solve the above-described problems on the helical scanning type X-ray CT imaging system, and therefore has an object to provide an X-ray CT imaging apparatus capable of improving the interpolation precision of the helical scanning image data and also an improved image data interpolating method in the helical scanning system.

To achieve the above-described object, an X-ray CT imaging apparatus (100), according to the present invention, comprises the features of claim 1.

A method for reconstructing tomographic images of multi-planar slices of an object under medical examination in an X-ray computerized tomographic imaging apparatus (100), according to the present invention, comprises the steps indicated in claim 5.

For a better understanding of the above-described present invention, reference is made to the following detailed description to be read in conjunction with the following drawings, in which:

Figs. 1A to 1C schematically illustrate one conventional X-ray CT imaging system for the third generation mode;
Figs. 2A to 2D schematically illustrate another conventional X-ray CT imaging system utilizing the helical scanning method;
Fig. 3 is a schematic block diagram of an overall arrangement of an X-ray CT imaging apparatus 100 according to a first preferred embodiment of the present invention;
Fig. 4 represents a basic idea of a full helical scanning type interpolation method executed in the imaging apparatus 100 shown in Fig. 3;
Figs. 5 and 6 are illustrations for pictorially explaining basic ideas of both projection data obtained by opposite X-ray beam and fan angle related to the X-ray CT imaging apparatus 100;

Fig. 7 is an illustration for explaining an interpolation method according to the present invention;

Fig. 8 is a schematic block diagram of an internal arrangement of the interpolation calculation unit 10 shown in Fig. 3;

Figs. 9A and 9B explain operations of the interpolation calculation unit 10; and,

Figs. 10A to 10C represents a half helical scanning type interpolation method in comparison with other interpolation methods.

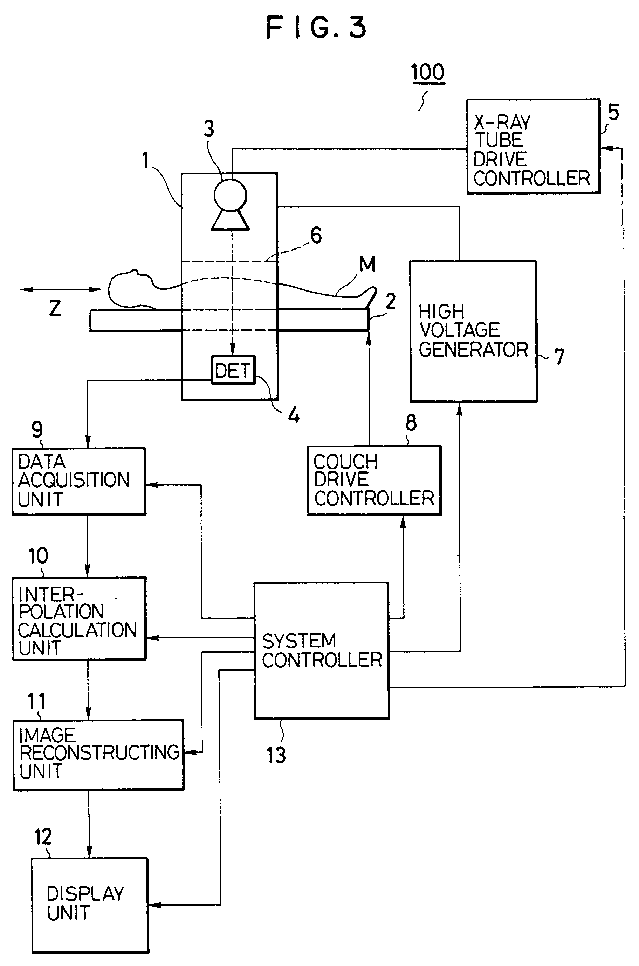

Referring now to Fig. 3, an overall arrangement of a helical scanning type X-ray CT (computerized tomographic) imaging apparatus 100 will be described.

In the helical scanning type X-ray CT imaging apparatus 100 shown in Fig. 3, a gantry 1 is provided with a dome 6 having a function to guard an object "M" under medical examination, e.g., a patient.

Within this gantry 1, an X-ray tube 3 is positioned opposite to an X-ray detector 4 with sandwiching the object "M". The X-ray tube 3 is electrically operated under control of a high voltage generator 7, and also is rotatably controlled around the dome 6 under control of an X-ray tube drive controller 5. The X-ray detector 4 comprises a plurality of single detectors that are positioned in an array form, and detect X-rays which have been generated from the X-ray tube 3 and penetrated through the object "M". The couch 2 is designed to be continuously translated along a longitudinal direction "Z" of the object "M" under medical examination under control of an couch drive controller 8. A data acquisition unit 9 for acquiring actual projection data detected by the X-ray detector 4 is connected to the X-ray detector 4. An interpolation calculating unit 10 is connected to this data acquisition unit 9 so as to perform an interpolation calculation for desirable (slice) projection data based on projection data produced by opposite X-ray beams (this will be discussed more in detail) according to the present invention, by utilizing the actually-acquired projection data from the data acquisition unit 9. An image reconstructing unit 11 is connected to this interpolation calculation unit 10 in order to execute an image reconstruction process based on the interpolated projection data sent from the interpolation calculation unit 10. A display unit 12 is connected to this image reconstructing unit 11, so that tomographic images of the object "M" under medical examination are displayed thereon in response to the image reconstruction data derived from the image reconstruction unit 11. Furthermore, a system controller 13 is employed in order to control all of the above-described circuit arrangements. This system controller 13 is mainly constructed of a central processing unit (CPU) (not shown in detail). In accordance with a feature of this preferred embodiment, the interpolation calculation unit 10 performs the below-mentioned interpolation calculation for the helical-scanned projection data under control of the system controller 13.

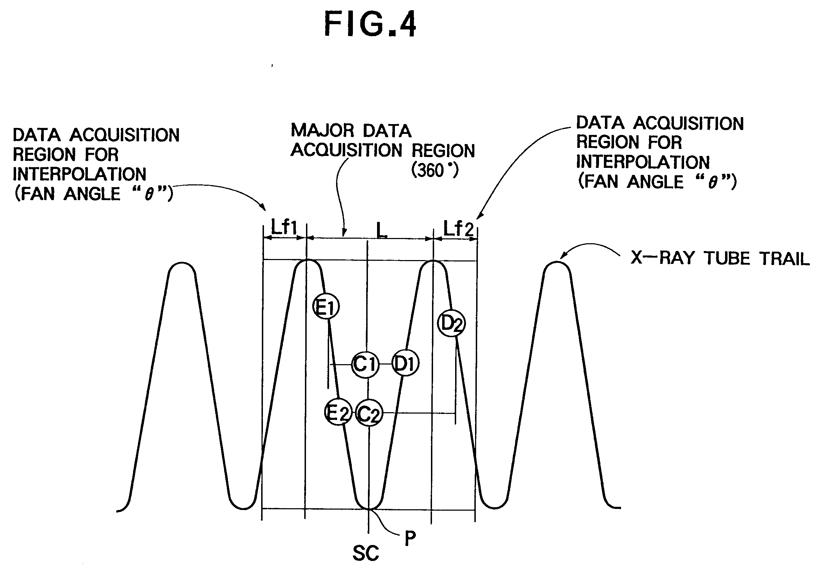

Fig. 4 illustrates a principal idea of a method for interpolating projection data acquired during the full helical scanning operation, in accordance with the present invention. In this figure, under such a condition that an X-ray tube trail is varied in a sinusoidal form, a region for obtaining a single slice, corresponding to 360° of an arbitrary one rotation of the X-ray tube 3 around the object "M" under examination, is set as a major data acquisition region "L", and also two data acquisition regions "$L_{f1}$" and "$L_{f2}$" for interpolation are set to both sides of the major data acquisition region "L", as viewed in Fig. 4. The maximum range of each data acquisition region "$L_{f1}$", "$L_{f2}$" for interpolation is selected to be one fan angle of the X-ray beam. Generally, the typical fan angle is designed from 35° to 70°.

Assuming now that slice projection data at a slice point "$C_1$" positioned on one slice plane is desired, the linear interpolation is carried out by utilizing both the actually-acquired projection data at a point "$D_1$" which is located nearest this slice point "$C_1$" and also has the same rotation phase relationship with this point "$C_1$" during the helical scanning (positioned on the X-ray tube trail), and the other actually-acquired projection data at a point "$E_1$" opposite to the above-described point "$D_1$" as a center of the slice center (SC), which is so-called "actually-acquired projection data obtained by an opposite X-ray beam" (simply referred to as "opposite-beam projection data"). Furthermore, when slice projection data at another slice point "$C_2$" is desired, the interpolation is similarly performed by utilizing both the actually-acquired projection data at a point "$E_2$" which is located nearest this slice point "$C_2$" and at the same rotation phase with this slice point "$C_2$" during the helical scanning, and also the other actually-acquired projection data at a point "$D_2$" (so-called "opposite-beam projection data") positioned opposite to the point "$E_2$" as a center of the slice center (SC). Such an interpolation calculation is executed by utilizing both the actually-acquired projection data and opposite-beam projection data with respect to all projection angles in the CT imaging apparatus according to the first preferred embodiment shown in Fig. 3.

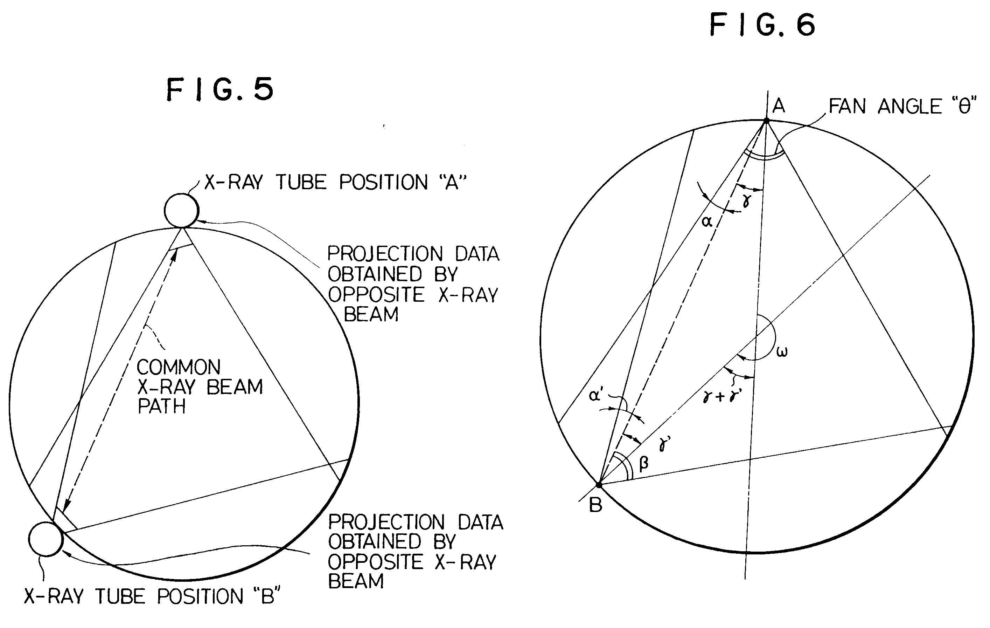

Fig. 5 schematically represents a definition of projection data obtained by an opposite X-ray fan beam.

It should be noted that since the X-ray detector (not shown) is positioned so as to receive the X-ray fan beam emitted from the X-ray tube 3 and penetrated through the object under medical examination (not shown either), it may be recognized that there are plural X-ray beam channels between the X-ray tube 3 and the detector, the quantity of which corresponds to the total element number of the X-ray detector.

Both a channel existing in projection data acquired at an X-ray tube position "A" and another channel present in projection data acquired at another X-ray tube position "B" located at the different rotation phase of this position "A", hold a common X-ray beam path, or channel as indicated by a dot line. In this case, there exists a so-called "relationship of projection data obtained by an opposite X-ray beam" between the projection data which has been acquired at a certain channel of the X-ray tube position "A" and the projection data which has been acquired at an arbitrary channel of the X-ray tube position "B" different from the first-mentioned channel of the X-ray tube position "B". These projection data are acquired under so-called "opposite X-ray beam relationship" within 360° range. As a consequence, in accordance with the so-termed "opposite beam interpolation" method of the present invention, desirable (slice) projection data at an arbitrary position along a center line of an arbitrary slice is calculated by employing actually acquired projection data which are present on both sides of this slice position and also at the same rotation phase with the arbitrary position, and further have the so-called "opposite beam relationship", so that the projection data interpolation may be performed by setting narrower data acquisition regions "$L_{f1}$" and "$L_{f2}$" than the conventional data acquisition regions "La" and "Lb" for interpolation.

In Fig. 6, there is shown a calculation sample using the opposite beam relationship according to the present invention.

It is assumed that a position of a certain detector channel of projection data from a point "A" is designated by an angle "$\alpha$" and an imaging position where there is "an opposite" X-ray beam which is positioned opposite to an X-ray beam corresponding to this detector channel, is designated by a point "B". At this time, an angle "$\beta$" for designating this channel position is defined by the following equation:

$$\beta = \Theta - \alpha' \qquad (2)$$

Furthermore, since $\alpha = \alpha'$ due to symmetry thereof, this angle "$\beta$" is defined as follows:

$$\beta = \Theta - \alpha \qquad (3)$$

On the other hand, another angle "$\omega$" is defined by the below-mentioned equation (4):

$$\begin{aligned}
\omega &= 180° + (\gamma + \gamma') \qquad (4)\\
&= 180° + 2\gamma \text{ (if } \gamma = \gamma')\\
&= 180° + 2\{(\Theta/2) - \alpha\}\\
&= 180° + \Theta - 2\alpha
\end{aligned}$$

It should be understood that this angle "$\omega$" is a rotation angle of the point "B" with reference to the point "A", and this rotation angle of the point "B" indicates a position of a fan-beam source (X-ray tube) involving an X-ray beam positioned opposite to an X-ray beam for a specific channel of the point "A".

When an opposite X-ray beam is represented by a channel number and projection data number in case of the 3rd generation mode X-ray imaging system, for instance, the opposite X-ray beam at an $\alpha$-th (No. $\alpha$) channel on the projection data at the point "A" will be expressed as follows:

Channel No. = No. $\beta$ = n - No. $\alpha$

Projection data No. ;

$$\text{No. } \omega = P \{1/2 + (n - 2 \text{ No. } \alpha)/2 \times \Theta/360\} \qquad (5)$$

where "n" is a channel number, "P" denotes a total number of projection data within one rotation, and No. $\omega$ is equal to P x $\omega$/360 .

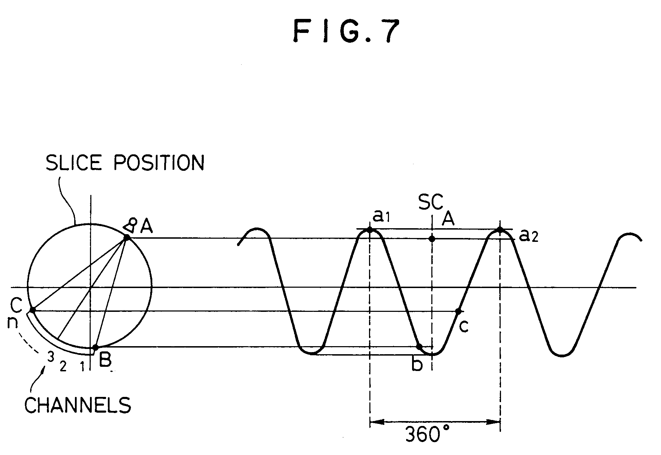

Referring now to Fig. 7, an overall interpolating sequence for helically-scanned projection data according to the first preferred embodiment will be summarized.

First, a slice position of a tomographic image to be reconstructed by the helical scanning operation is set. Thereafter, a search is performed for X-ray beams positioned opposite with respect to the slice position as to each channel at the overall projection position on the slice position.

When slice projection data, for instance, "A" on the slice position shown in Fig. 7 is acquired, "n" pieces (channels) of virtually-existing X-ray beams from a first line (channel) $\overline{AB}$ to a last line (channel) $\overline{AC}$ are used for the linear interpolation. It should be noted that a virtually-existing X-ray beam connecting the points "A" and "C" is obtained from an n-th path at a position "$a_1$" located on the X-ray tube trail and a first path at a position "c" (corresponding to the opposite X-ray beam with respect to this virtually-existing X-ray beam). Similarly, another virtually-existing X-ray beam connecting the points "A" and "B" is obtained from a first path at a position "$a_2$" located on the X-ray tube trail and an n-th path at a position "b" (corresponding to the opposite X-ray beam with respect to this virtually existing X-ray beam).

Subsequently, the linear interpolation is carried out by employing the respective projection data produced by the mutually opposite X-ray beams which have been obtained for the respective channels, as explained above, so that desirable slice projection data at the slice position "A" can be obtained. Furthermore, other different projection data are similarly obtained, whereby all of the projection data at the slice positions may be interpolated.

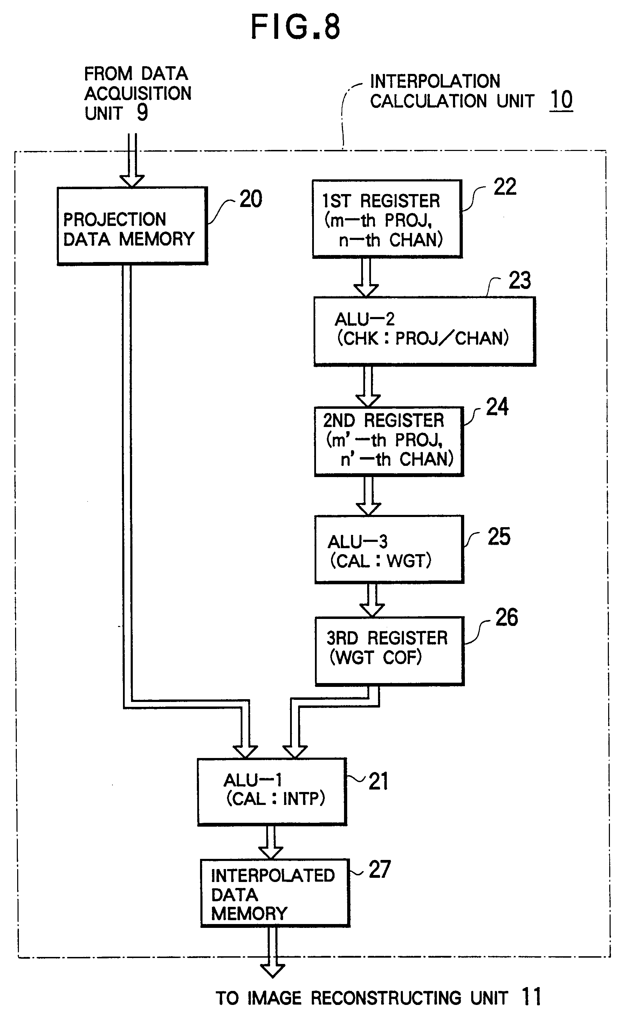

In Fig. 8, there is shown an internal arrangement of the interpolation calculation unit 10 represented in Fig. 3. It should be noted that for the sake of simplicity, no indication is made of the system controller 13 for controlling all of these internal arrangements.

The interpolation calculation unit 10 shown in Fig. 8 includes a projection data memory 20 for receiving the actually-acquired projection data from the data acquisition unit 9 represented in Fig. 3, and for storing these projection data therein. These actually-acquired projection data stored in this memory 20 will be furnished to a first arithmetic and logic unit (ALU-1) 21 for the purpose of linear interpolation calculation. The interpolation calculation unit 10 further includes a first register 22 into which both a projection number (e.g., n-th projection number) and a channel number (e.g.. n-th channel) have been stored in order to designate slice projection data to be interpolated; a second ALU 23 for checking whether or not opposite-beam projection data is present at an opposite side with respect to a slice center (plane) and also for obtaining both opposite-beam projection data (e.g., m'-th projection data) and a channel number thereof (e.g., n'-th channel number); and also a second register 24 for storing both the opposite-beam projection data and channel number thereof obtained by the second ALU 23. The interpolation calculation unit 10 further includes a third ALU 25 for calculating weight coefficients based upon the opposite-beam projection data and channel number thereof; and a third register 26 for storing the weight coefficients calculated by the second ALU 25. Thus, the calculated weight coefficients are supplied to the above-described first ALU 21 so as to perform the linear interpolation calculation with respect to the designated slice projection data, while utilizing the actually-acquired projection data stored in the projection data memory 20 and also the opposite-beam projection data based on the weight coefficients. Then, the interpolated-projection data is stored in an interpolated data memory 27 employed in this interpolation calculation unit 10.

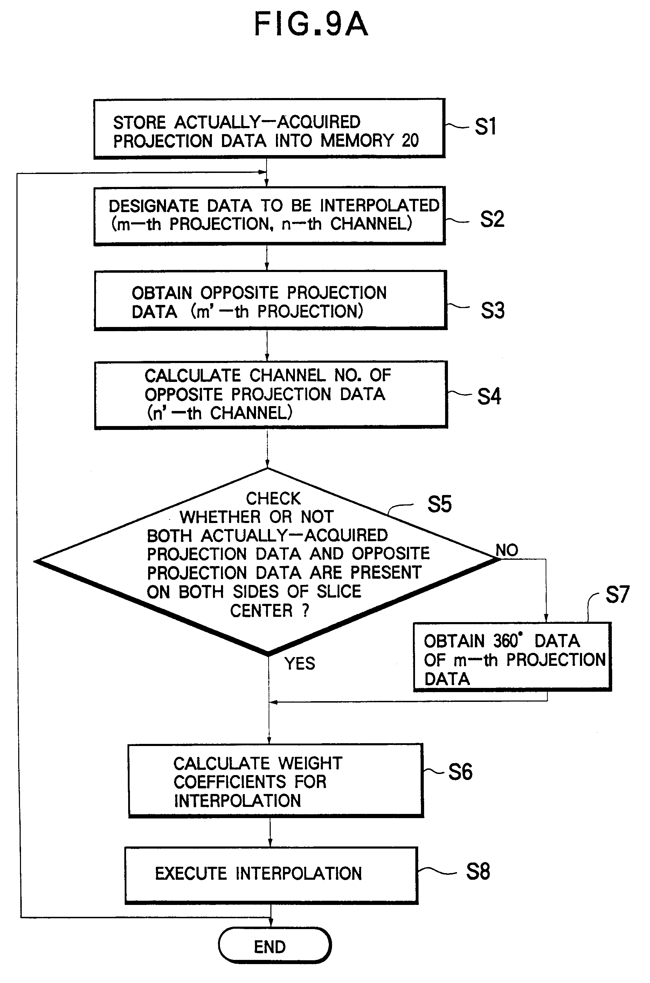

An interpolation calculating sequence of the interpolation calculation unit 10 will now be described with reference to Fig. 9A.

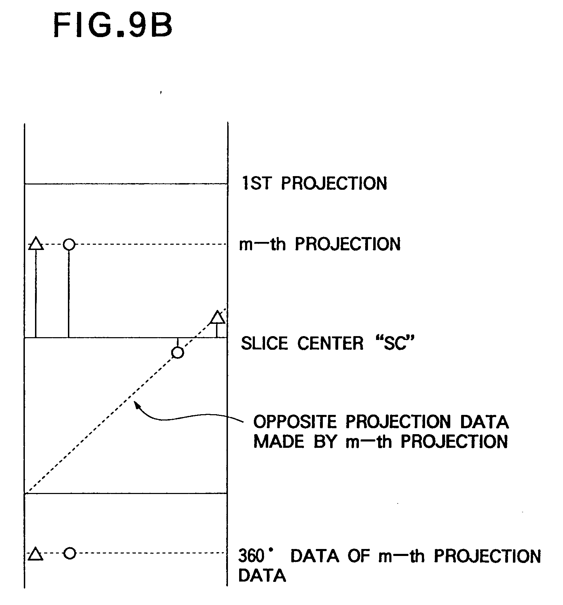

In the flowchart shown in Fig. 9, the actually-acquired projection data is stored into the projection data memory 20 at a first step S1. Thereafter, the data to be interpolated (i.e., slice projection data) is designated by utilizing, for instance, the m-th projection number and n-th channel number at a step S2. At a next step S3, the opposite-beam projection data (e.g., m'-th projection number) is calculated. Subsequently, the channel number (e.g., n'-th channel number) of the opposite-beam projection data is calculated at a step S4. At a next step S5, a check is made whether or not both the actually-acquired projection data and opposite-beam projection data are present on both sides of the slice center. If YES, then the weight coefficients for the opposite-beam projection data and actually-acquired projection data are calculated in the third ALU 25 at a step S6. To the contrary, if both data are not present on both sides of the slice center at the previous step S7, 360° data of the projection data to be interpolated (e.g., m-th projection data) is obtained at a step S7. Then, based on the 360° data, weight coefficients are similarly calculated at the step S6. Subsequently, an interpolation calculation is executed for the projection data to be interpolated by utilizing the calculated weight coefficients at a step 8. A similar projection data designation and interpolation calculation is carried out for the remaining projection data present on the slice center from the step S2 until the step S8.

Fig. 9A is an illustration for explaining why the above-described checking step S5 shown in the above flowchart is required.

In case of, for instance, the slice projection data located on the slice plane and at the same rotation phase with the m-th projection data (actually acquired), the opposite-beam projection data is obtained with respect to a specific channel of the m-th projection data and a check must be done whether or not this opposite-beam projection data is located at an opposite side with respect to the side where the m-th projection data is present. That is, one opposite-beam projection data indicated by a symbol "o" is located at the opposite side as a center of the slice center. Conversely, another opposite-beam projection data indicated by a symbol "Δ" is located at the same side where the m-th projection data is present. In the latter case, since this opposite-beam projection data indicated by the symbol "Δ" is not directly utilized for the interpolation calculation, 360° data of the m-th projection data is obtained for the linear interpolation calculation, because this projection data is located at the same side as the m-th projection data.

As previously described in the first preferred embodiment, slice projection data corresponding to an arbitrary rotation phase on an arbitrary slice plane may be interpolated by employing the so-called "opposite-beam projection data" and such a data interpolation may be realized by setting the narrow data regions $L_{f1}$ and $L_{f2}$ for interpolation purposes. As a consequence, a thickness of an effective slice image may be considerably reduced, as compared with that of the conventional interpolation method. Consequently, since the actually-acquired projection data obtained from a position which is relatively short from the slice plane may be utilized for this interpolation calculation, namely this interpolated projection data substantially becomes true projection data, precision of the interpolation calculation may be improved.

In the above-described first interpolating method, a range of the major data acquisition region "L" was selected to be 360°. The present invention is not limited to such a full-helical scanning method, but may be a half-helical scanning method.

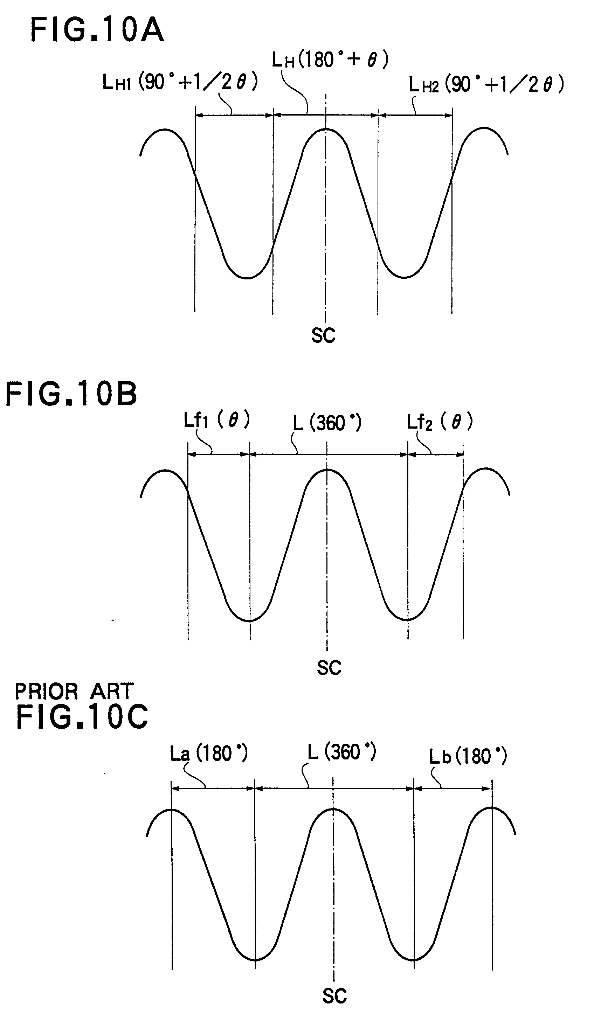

For a better understanding of the merits achieved by the half-helical scanning method, there are represented; two data acquisition regions "$L_{H1}$" and "$L_{H2}$" for interpolation (each 90° + 1/2 Θ) used for the half-helical scanning method in Fig. 10A, in comparison with the data acquisition regions "$L_{f1}$" and "$L_{f2}$" (each Θ) for the full-helical scanning method of the first preferred embodiment in Fig. 10B, and also the data acquisition regions "$L_a$" and "$L_b$" (each 180°) for the conventional helical scanning method in Fig. 10C.

As apparent from these figures 10A to 10C, since a thickness of an effective slice image may be furthermore reduced in accordance with the half-helical scanning method represented in Fig. 10A, precision of the interpolation calculation can be further improved as compared with that of the first preferred embodiment.

Although the above-described slice projection data interpolation methods are performed by utilizing two projection data, i.e., actually-acquired projection data of one point on the slice plane and also opposite-beam projection data of the other point thereon, a high-order interpolation method (e.g., Lagrange's interpolation) with employment of projection data of more than two points on the same slice plane may be utilizied in the present invention. Moreover, the above-described preferred embodiments are realized in the 3rd generation type X-ray CT imaging system. The present invention may be apparently realized also in the 4th generation type X-ray CT imaging system.

**Claims**

1. An X-ray computerized tomographic imaging apparatus (100) for reconstructing tomographic images of multi-planar slices of an object under medical examination, comprising:

    table couch means (2) for supporting the object (M) under medical examination;
    table couch drive control means (8) for controlling a continuous transportation of the table couch means (2) along a longitudinal axis of the object (M);
    an X-ray beam source (3) for radiating a fan-shaped X-ray beam toward the multi-planar slices of the object (M);
    detector means (4) for detecting the fan-shaped X-ray beam which has penetrated through the slices of the objects (M) to thereby produce projection data representative of profile intensities of the fan-shaped X-ray beam;
    means (1) for mounting both the X-ray beam source (3) and the detector means (4) in such a manner that the X-ray beam source (3) is continuously positioned opposite to the detector means (4) with respect to the slice of the object (M), while detecting the fan-shaped radiation beam, and for performing a relative movement between the X-ray beam source (3) and the object (M) in a plane which is perpendicular to the longitudinal axis of the object (M), whereby a helical scanning around the object (M) is realized by the continuous transportation of the table couch means (2) and the relative movement between them;
    data acquisition means for acquiring projection data obtained through a gantry rotation;
    interpolation calculating means (10) for obtaining slice projection data at a predetermined slice point ($C_1$:$C_2$) on a slice plane (SC) on the basis of data from a major data acquisition region (L; $L_H$) and two data acquisition regions for interpolation ($L_{f1}$, $L_{f2}$; $L_{H1}$, $L_{H2}$), and means (11) for reconstructing a tomographic image of the object,

    characterized in that

said interpolation calculating means (10) obtains the slice projection data at a predetermined slice point ($C_1$: $C_2$) by performing an interpolation calculation based upon at least one pair of first actually-acquired projection data at a first point ($D_1$:$E_2$) which is located nearest the slice point ($C_1$:$C_2$) and has the same rotation phase relationship with the slice point ($C_1$:$C_2$) during the helical scanning, and also second actually-acquired projection data at a second point ($E_1$:$D_2$) located opposite to the first point ($D_1$:$E_2$) as a center of the slice plane (SC); each data acquisition region for interpolation ($L_{f1}$, $L_{f2}$; $L_{H1}$, $L_{H2}$) corresponding to an angle of less than 180°.

2. An X-ray computerized tomographic imaging apparatus (100) as claimed in claim 1, wherein said interpolation calculating means (10) executes the linear interpolation method based upon said one pair of actually-acquired projection data.

3. An X-ray computerized tomographic imaging apparatus (100) as claimed in claim 1, wherein said interpolation calculating means (10) executes the Lagrange's interpolation method based upon at least two different pairs of actually-acquired projection data.

4. An X-ray computerized tomographic imaging apparatus (100) as claimed in claim 1, wherein said interpolation calculating means (10) includes:

    means (22) for designating said slice projection data;
    means (23:24) for obtaining the second actually-acquired projection data at the second point ($E_1$:$D_2$) located opposite to the first point ($D_1$:$E_2$) as the center of the slice plane (SC) based on said designated slice projection data;
    means (25) for calculating weight coefficients for said one pair of first and second actually-acquired projection data at the first point ($D_1$:$E_2$) and second point ($E_1$:$D_2$) based upon a positional relationship between said first and second points with respect to said slice plane (SC);
    means (21) for performing the interpolation calculation by utilizing both of said first and second actually-acquired projection data at the first and second points and also the calculated weight coefficients thereof.

5. A method for reconstructing tomographic images of multi-planar slices of an object under medical examination in an X-ray computerized tomographic imaging apparatus (100), comprising the steps of:

radiating the object (M) under medical examination by a fan-shaped X-ray beam, while continuously translating the object (M) along a longitudinal axis thereof and relatively rotating an X-ray radiating source (3) and a detector (4) for detecting the fan-shaped X-ray beam penetrated through the object to produce projection data representative of profile intensities of the fan-shaped X-ray beam, whereby a helical scanning around the object (M) is realized by the continuous transportation of the object (M);

executing an interpolation calculation so as to obtain slice projection data at a predetermined slice ($C_1:C_2$) on a slice plane (SC) on the basis of data from a major data acquisition region (L; $L_H$) and two data acquisition regions for interpolation ($L_{f1}$, $L_{f2}$; $L_{H1}$, $L_{H2}$), and

reconstructing a tomographic image of the object form the data,

characterized by said executing step comprising the step of

utilizing at least one pair of first actually-acquired projection data at a first point ($D_1:E_2$) which is located nearest the slice point ($C_1:C_2$) and has the same rotation phase relationship with the slice point ($C_1:C_2$) during the helical scanning, and also second actually-acquired projection data at a second point ($E_1:D_2$) located opposite to the first point ($D_1:E_2$) as a center of the slice plane (SC), each data acquisition region for interpolation ($L_{f1}$, $L_{f2}$; $L_{H1}$, $L_{H2}$) corresponding to an angle of less than 180°.

6. A method as claimed in claim 5, wherein said interpolation calculation is the linear interpolation method by utilizing said one pair of first and second actually-acquired projection data.

7. A method as claimed in claim 5, wherein said interpolation calculation is the Lagrange's interpolation method by utilizing at least two different pairs of actually-acquired projection data.

**Patentansprüche**

1. Computerisiertes tomographisches Röntgenstrahl-Abbildungsgerät (100) für die Rekonstruktion von tomographischen Bildern von mehrere Ebenen enthaltenden Scheiben eines Objekts, das sich in medizinischer Untersuchung befindet, mit:

einer Tischbetteinrichtung (2) für die Lagerung des Objekts (M), das sich in medizinischer Untersuchung befindet, einer Steuereinrichtung (8) für den Antrieb der Tischbetteinrichtung, die zur Steuerung eines kontinuierlichen Transports der Tischbetteinrichtung (2) entlang einer Längsachse des Objekts (M) ausgelegt ist,

einer Röntgenstrahlquelle (3) für die Aussendung eines fächerförmigen Röntgenstrahls in Richtung zu den mehrere Ebenen enthaltenden Scheiben des Objekts (M),

einer Detektoreinrichtung (4) für die Erfassung des fächerförmigen Röntgenstrahls, der durch die Scheiben des Objekts (M) hindurchgegangen ist, um hierdurch Projektionsdaten zu erzeugen, die für Intensitäten des Profils des fächerförmigen Röntgenstrahls repräsentativ sind,

einer Einrichtung (1) zum Anbringen bzw. Halten sowohl der Röntgenstrahlquelle (3) als auch der Detektoreinrichtung (4) in einer solchen Weise, daß die Röntgenstrahlquelle (3) der Detektoreinrichtung (4) unter Bezug auf die Scheibe des Objekts (M) durchgehend gegenüberliegend positioniert ist, wobei hierbei der fächerförmige Strahl erfaßt wird, und zum Durchführen einer relativen Bewegung zwischen der Röntgenstrahlquelle (3) und dem Objekt (M) in einer Ebene, die rechtwinklig zu der Längsachse des Objekts (M) verläuft, wodurch eine schraubenlinienförmige Abtastung um das Objekt (M) herum durch den kontinuierlichen Transport der Tischbetteinrichtung (2) und die relative Bewegung zwischen diesen Komponenten realisiert wird,

einer Datengewinnungseinrichtung zur Gewinnung von Projektionsdaten, die durch eine Drehung eines Stützgerüsts erhalten werden,

einer Interpolationsberechnungseinrichtung (10) für die Bildung von Scheibenprojektionsdaten an einem vorbestimmten Scheibenpunkt ($C_1:C_2$) in einer Scheibenebene (SC) auf der Grundlage von Daten, die von einer hauptsächlichen Datengewinnungsregion (L; $L_H$) und zwei Datengewinnungsregionen für eine Interpolation ($L_{f1}$, $L_{f2}$; $L_{H1}$, $L_{H2}$) stammen, und

einer Einrichtung (11) für die Rekonstruktion eines tomographischen Bilds des Objekts,

dadurch **gekennzeichnet,** daß

die Interpolationsberechnungseinrichtung (10) die Scheibenprojektionsdaten an einem vorbestimmten Scheibenpunkt ($C_1:C_2$) dadurch erhält, daß eine Interpolationsberechnung auf der Grundlage von mindestens einem Paar von ersten, aktuell erhaltenen Projektionsdaten an einem ersten Punkt ($D_1:E_2$), der am nächsten bei dem Scheibenpunkt ($C_1:C_2$) angeordnet ist und die gleiche Drehphasenbeziehung bezüglich des Scheibenpunkts ($C_1:C_2$) während der schraubenlinienförmigen Abtastung aufweist, und ferner zweiten, aktuell gewonnenen Projektionsdaten an einem zweiten Punkt ($E_1:D_2$)

durchführt, der dem ersten Punkt ($D_1$:$E_2$) gegenüberliegt, wobei die Scheibenebene (SC) als ein Zentrum dient, wobei jede Datengewinnungsregion für die Interpolation ($L_{f1}$, $L_{f2}$; $L_{H1}$, $L_{H2}$) einem Winkel von weniger als 180° entspricht.

2. Computerisiertes tomographisches Röntgenstrahl-Abbildungsgerät (100) nach Anspruch 1, bei dem die Interpolationsberechnungseinrichtung (10) die lineare Interpolationsmethode auf der Grundlage des besagten einen Paares der aktuell gewonnenen Projektionsdaten durchführt.

3. Computerisiertes tomographisches Röntgenstrahl-Abbildungsgerät (100) nach Anspruch 1, bei dem die Interpolationsberechnungseinrichtung (10) die Lagrange'sche Interpolationmethode auf der Grundlage von mindestens zwei unterschiedlichen Paaren von aktuell gewonnenen Projektionsdaten durchführt.

4. Computerisiertes tomographisches Röntgenstrahl-Abbildungsgerät (100) nach Anspruch 1, bei dem die Interpolationsberechnungseinrichtung (10) aufweist:

eine Einrichtung (22) zum Bezeichnen bzw. Bestimmen der Scheibenprojektiondaten,
eine Einrichtung (23:24) für den Erhalt der zweiten, aktuell gewonnenen Projektionsdaten an dem zweiten Punkt ($E_1$:$D_2$) auf der Basis der bezeichneten Scheibenprojektionsdaten, wobei der zweite Punkt dem ersten Punkt ($D_1$:$E_2$) unter Bezugnahme auf das Zentrum der Scheibenebene (SC) gegenüberliegend angeordnet ist,
eine Einrichtung (25) für die Berechnung von Gewichtskoeffizienten für das besagte eine Paar der ersten und zweiten, aktuell gewonnenen Projektionsdaten an dem ersten Punkt ($D_1$:$E_2$) und dem zweiten Punkt ($E_1$:$D_2$) auf der Grundlage einer positionsmäßigen Beziehung zwischen dem ersten und dem zweiten Punkt unter Bezugnahme auf die Scheibenebene (SC),
eine Einrichtung (21) für die Durchführung der Interpolationsberechnung unter Heranziehung sowohl der ersten als auch der zweiten aktuell gewonnenen Projektionsdaten an dem ersten und dem zweiten Punkt und auch deren berechneten Gewichtskoeffizienten.

5. Verfahren zum Rekonstruieren von tomographischen Bildern von mehrere Ebenen enthaltenen Scheiben eines Objekts, das sich unter medizinischer Untersuchung befindet, in einem computerisierten, tomographischen Röntgenstrahl-Abbildungsgerät (100), mit den Schritten:

Bestrahlen des Objekts (M), das sich in medizinischer Untersuchung befindet, mit Hilfe eines fächerförmigen Röntgenstrahls, wobei das Objekt (M) kontinuierlich entlang einer Längsachse desselben translatorisch bewegt wird und eine Röntgenstrahlquelle (3) und ein Detektor (4) für die Erfassung des fächerförmigen Röntgenstrahls, der durch das Objekt hindurchgegangen ist, relativ gedreht werden, um hierdurch Projektionsdaten zu erzeugen, die für Intensitäten des Profils des fächerförmigen Röntgenstrahls repräsentativ sind, wodurch eine schraubenlinienförmige Abtastung um das Objekt (M) herum durch den kontinuierlichen Transport des Objekts (M) erzielt wird,
Ausführen einer Interpolationsberechnung derart, daß Scheibenprojektionsdaten an einer vorbestimmten Scheibe ($C_1$:$C_2$) auf einer Scheibenebene (SC) erhalten werden, und zwar auf der Grundlage von Daten aus einer hauptsächlichen Datengewinnungsregion (L; $L_H$) und zwei Datengewinnungsregionen für die Interpolation ($L_{f1}$, $L_{f2}$; $L_{H1}$, $L_{H2}$), und
Rekonstruieren eines tomographischen Bilds des Objekts aus den Daten,

dadurch **gekennzeichnet,** daß der Ausführungsschritt den Schritt enthält:

Benutzen von mindestens einem Paar von ersten, aktuell gewonnenen Projektionsdaten an einem ersten Punkt ($D_1$:$E_2$), der am nächsten bei dem Scheibenpunkt ($C_1$:$C_2$) benachbart ist und die gleiche Drehphasenbeziehung mit dem Scheibenpunkt ($C_1$:$C_2$) während der schraubenlinienförmigen Abtastung aufweist, und auch von zweiten, aktuell gewonnenen Projektionsdaten an einem zweiten Punkt ($E_1$:$D_2$), der dem ersten Punkt ($D_1$:$E_2$) als eine Mitte der Scheibenebene (SC) oder bezüglich dieser Scheibenebenenmitte gegenüberliegend angeordnet ist, wobei jede Datengewinnungsregion für die Interpolation ($L_{f1}$, $L_{f2}$; $L_{H1}$, $L_{H2}$) einem Winkel von weniger als 180° entspricht.

6. Verfahren nach Anspruch 5, bei dem die Interpolationsberechnung die lineare Interpolationsmethode ist, bei der das eine Paar der ersten und zweiten, aktuell gewonnenen Projektionsdaten herangezogen wird.

7. Verfahren nach Anspruch 5, bei dem die Interpolationsberechnung die Lagrange'sche Interpolationsmethode ist, bei der mindestens zwei unterschiedliche Paare von aktuell gewonnenen Projektionsdaten benutzt werden.

## Revendications

1. Appareil (100) d'imagerie de rayons X par tomographie informatisée, permettant de reconstruire des images tomographiques de coupes formées dans plusieurs plans d'un objet soumis à un examen médical, comprenant :

   un moyen (2) formant un lit de table et destiné à soutenir le sujet (M) soumis à l'examen médical;
   un moyen (8) de commande d'entraînement de lit de table, servant à commander le transport continu du moyen (2) formant un lit de table suivant l'axe longitudinal du sujet (M);
   une source (3) de faisceau de rayons X servant à émettre un faisceau de rayons X en forme d'éventail en direction des coupes formées dans plusieurs plans du sujet (M);
   un moyen détecteur (4) servant à détecter le faisceau de rayons X en forme d'éventail qui a pénétré dans les coupes du sujet (M) de manière à produire des données de projection représentatives d'intensités de profils du faisceau de rayons X en forme d'éventail;
   un moyen (1) servant à monter à la fois la source (3) de faisceau de rayons X et le moyen détecteur (4) de manière que la source (3) de faisceau de rayons X soit continûment positionnée à l'opposé du moyen détecteur (4) par rapport à la coupe du sujet (M), pendant la détection du faisceau de rayonnement en forme d'éventail, et à réaliser un déplacement relatif entre la source de faisceau de rayons X (3) et le sujet (M) dans un plan qui est perpendiculaire à l'axe longitudinal du sujet (M), si bien qu'une analyse en hélice entourant le sujet (M) est réalisée par le transport continu du moyen (2) formant un lit de table et le mouvement relatif existant entre eux;
   un moyen d'acquisition de données servant à acquérir des données de projection obtenues par l'intermédiaire de la rotation d'un portique;
   un moyen (10) de calcul d'interpolation permettant d'obtenir des données de projection de coupe au niveau d'un point d'une coupe prédéterminée ($C_1$:$C_2$) sur le plan d'une coupe (SC) sur la base de données provenant d'une région principale d'acquisition de données (L ; $L_H$) et de deux régions d'acquisition de données relatives à l'interpolation ($L_{f1}$, $L_{f2}$ ; $L_{H1}$, $L_{H2}$), et
   un moyen (11) permettant de reconstruire une image tomographique du sujet,

   caractérisé en ce que:

   ledit moyen (10) de calcul d'interpolation obtient les données de projection de coupe en un point prédéterminé de la coupe ($C_1$:$C_2$) en effectuant un calcul d'interpolation sur la base d'au moins une paire de données formées d'une première donnée de projection réellement acquise en un premier point ($D_1$:$E_2$), qui est placé le plus près du point de la coupe ($C_1$:$C_2$) et possède la même relation de phase de rotation avec le point de la coupe ($C_1$:$C_2$) pendant l'analyse en hélice, et d'une deuxième donnée de projection réellement acquise en un deuxième point ($E_1$:$D_2$) placé à l'opposé du premier point ($D_1$:$E_2$), faisant fonction de centre du plan de la coupe (SC) ; chaque région d'acquisition de données relative à l'interpolation ($L_{f1}$, $L_{f2}$ ; $L_{H1}$, $L_{H2}$) correspondant à un angle inférieur à 180°.

2. Appareil (100) d'imagerie de rayons X par tomographie informatisée selon la revendication 1, où ledit moyen (10) de calcul d'interpolation exécute le procédé d'interpolation linéaire en s'appuyant sur ladite paire de données de projection réellement acquises.

3. Appareil (100) d'imagerie de rayons X par tomographie informatisée selon la revendication 1, où ledit moyen (10) de calcul d'interpolation exécute le procédé d'interpolation de Lagrange en s'appuyant sur au moins deux paires différentes de données de projection réellement acquises.

4. Appareil (100) d'imagerie de rayons X par tomographie informatisée selon la revendication 1, où ledit moyen (10) de calcul d'interpolation comporte :

   un moyen (22) servant à désigner lesdites données de projection de coupe ;
   un moyen (23:24) servant à obtenir la deuxième donnée de projection réellement acquise au deuxième point ($E_1$:$D_2$) placé à l'opposé du premier point ($D_1$:$E_2$), faisant fonction du centre du plan de la coupe (SC) sur la base desdites données de projection de coupe désignées ;
   un moyen (25) servant à calculer des coefficients de pondération pour ladite paire formée desdites première et deuxième données de projection réellement acquises au premier point ($D_1$:$E_2$) et au deuxième point ($E_1$:$D_2$) sur la base de la relation de position existant entre lesdits premier et deuxième points par rapport audit plan de la coupe (SC);
   un moyen (21) permettant d'effectuer le calcul d'interpolation en utilisant à la fois lesdites première et deuxième données de projection réellement acquises aux premier et deuxième points et aussi leurs coefficients de pondération calculés.

5. Procédé de reconstruction d'images tomographiques de coupes formées dans plusieurs plans d'un sujet soumis à un examen médical dans un appareil (100) d'imagerie de rayons X par tomographie informatisée, comprenant les opérations suivantes :

irradier le sujet (M) soumis à l'examen médical au moyen d'un faisceau de rayons X en forme d'éventail, tout en déplaçant de façon continue l'objet (M) sur son axe longitudinal et en faisant relativement tourner une source (3) de rayonnement de rayons X et un détecteur (4) servant à détecter le faisceau de rayons X en forme d'éventail qui a pénétré à travers le sujet de façon à produire des données de projection représentatives d'intensités de profils du faisceau de rayons X en forme d'éventail, de sorte qu'une analyse en hélice autour du sujet (M) est réalisée par le transport continu du sujet (M);
exécuter un calcul d'interpolation de façon à obtenir des données de projection de coupe en une coupe prédéterminée ($C_1$:$C_2$) sur un plan de la coupe (SC) sur la base de données venant d'une région principale d'acquisition de données (L ; LH) et de deux régions d'acquisition de données relatives à l'interpolation ($L_{f1}$, $L_{f2}$ ; $L_{H1}$, $L_{H2}$), et
reconstruire une image tomographique du sujet à partir des données,

caractérisé en ce que ladite opération d'exécution comprend l'opération suivante :

utiliser au moins une paire de données formée d'une première donnée de projection réellement acquise en un premier point ($D_1$:$E_2$) qui est placé le plus près du point de la coupe ($C_1$:$C_2$) et possède la même relation de phase de rotation avec le point de la coupe ($C_1$:$C_2$) pendant l'analyse en hélice, et d'une deuxième donnée de projection réellement acquise en un deuxième point ($E_1$:$D_2$) placé à l'opposé du premier point ($D_1$:$E_2$) faisant fonction de centre du plan de la coupe (SC), chaque région d'acquisition de données relative à l'interpolation ($L_{f1}$, $L_{f2}$ ; $L_{H1}$, $L_{H2}$) correspondant à un angle inférieur à 180°.

6. Procédé selon la revendication 5, où ledit calcul d'interpolation est le procédé d'interpolation linéaire où l'on utilise ladite paire de première et deuxième données de projection réellement acquises.

7. Procédé selon la revendication 5, où ledit calcul d'interpolation est le procédé d'interpolation de Lagrange où l'on utilise au moins deux paires différentes de données de projection réellement acquises.

# FIG. 1A

# FIG. 1B

# FIG. 1C

EP 0 450 152 B1

PRIOR ART
F I G. 2A

PRIOR ART
F I G. 2B

PRIOR ART
F I G. 2C

POSITIONAL
SHIFT

360°  0°

SLICE
IMAGE

XB

M

3

2

4

K

M

EP 0 450 152 B1

**PRIOR ART**

# F I G. 2D

MAJOR DATA ACQUISITION
REGION ( 360° )

DATA ACQUISITION
REGION FOR INTERPOLATION
( 180° )

DATA ACQUITION REGION
FOR INTERPOLATION
(180° )

X-RAY TUBE
TRAIL

La | L | Lb

B₁   C₁   A₁

A₂ C₂   B₂

SC   P

# F I G. 3

# FIG.4

DATA ACQUISITION REGION FOR INTERPOLATION (FAN ANGLE "$\theta$")

MAJOR DATA ACQUISITION REGION (360°)

DATA ACQUISITION REGION FOR INTERPOLATION (FAN ANGLE "$\theta$")

Lf1  L  Lf2

X–RAY TUBE TRAIL

E1

D2

C1  D1

E2  C2

P

SC

EP 0 450 152 B1

FIG. 6

FIG. 5

# FIG. 7

SLICE POSITION

CHANNELS

SC

360°

# FIG.8

FROM DATA
ACQUISITION
UNIT 9

INTERPOLATION
CALCULATION UNIT 10

PROJECTION
DATA MEMORY — 20

1ST REGISTER
(m—th PROJ,
n—th CHAN) — 22

ALU—2
(CHK : PROJ/CHAN) — 23

2ND REGISTER
(m'—th PROJ,
n'—th CHAN) — 24

ALU—3
(CAL : WGT) — 25

3RD REGISTER
(WGT COF) — 26

ALU—1
(CAL : INTP) — 21

INTERPOLATED
DATA
MEMORY — 27

TO IMAGE RECONSTRUCTING UNIT 11

# FIG.9A

STORE ACTUALLY—ACQUIRED PROJECTION DATA INTO MEMORY 20 — S1

DESIGNATE DATA TO BE INTERPOLATED (m—th PROJECTION, n—th CHANNEL) — S2

OBTAIN OPPOSITE PROJECTION DATA (m'—th PROJECTION) — S3

CALCULATE CHANNEL NO. OF OPPOSITE PROJECTION DATA (n'—th CHANNEL) — S4

CHECK WHETHER OR NOT BOTH ACTUALLY—ACQUIRED PROJECTION DATA AND OPPOSITE PROJECTION DATA ARE PRESENT ON BOTH SIDES OF SLICE CENTER ? — S5

NO

OBTAIN 360° DATA OF m—th PROJECTION DATA — S7

YES

CALCULATE WEIGHT COEFFICIENTS FOR INTERPOLATION — S6

EXECUTE INTERPOLATION — S8

END

# FIG.9B

1ST PROJECTION

m—th PROJECTION

SLICE CENTER "SC"

OPPOSITE PROJECTION DATA
MADE BY m—th PROJECTION

360° DATA OF m—th PROJECTION
DATA

## FIG.10A

$L_{H1}(90° + 1/2θ)$  $L_H(180° + θ)$  $L_{H2}(90° + 1/2θ)$

SC

## FIG.10B

$Lf_1(θ)$  $L(360°)$  $Lf_2(θ)$

SC

PRIOR ART
## FIG.10C

$La(180°)$  $L(360°)$  $Lb(180°)$

SC